# DEMANDE DE BREVET EUROPEEN

(11) **EP 0 945 461 A1**
(43) Date de publication de la demande: **29.09.1999**
(21) Numéro de dépôt: 99105773.8
(22) Date de dépôt: 18.12.1991
(51) Int. Cl.: C07K 14/16, A61K 47/48

(54) **Lipopeptides inducteurs des lymphocytes T cytotoxiques et utilisation comme vaccins**

(30) Priorité: 18.12.1990 FR 9015870
(62) Demande divisionnaire de: 91403446.7
(71) Demandeur: INSTITUT PASTEUR DE LILLE, 59019 Lille Cédex (FR); INSTITUT NATIONAL DE LA SANTE ET DE LA RECHERCHE MEDICALE (INSERM), 75654 Paris Cédex 13 (FR)
(72) Inventeur: Boutillon, Christophe, 59800 Lille (FR); Martinon, Frédéric, 92120 Montrouge (FR); Sergheraert, Christian, 59190 Morbecque (FR); Magne Rémy, 78620 L'Etang la Ville (FR); Gras-Masse, Hélèna, 59710 Merignies (FR); Gomard, Elisabeth, 75017 Paris (FR); Tartar, André, 62490 Vitry en Artois (FR); Levy, Jean-Paul, 75013 Paris (FR)
(74) Mandataire: Phélip, Bruno

(57) **Abrégé**

Lipopeptides inducteurs des lymphocytes cytotoxiques comprenant une partie peptidique ayant entre 10 et 40 acides aminés environ et comportant au moins un déterminant antigénique induisant spécifiquement des lymphocytes T cytotoxiques. Les lipopeptides comprennent également une ou plusieurs chaînes dérivées d'acides gras choisis parmi l'acide 2-aminohexadécanoïque, le pimélautide et le triméxautide et/ou un ou plusieurs groupements stéroïdes modifiés.

Lesdits lipopeptides peuvent être utilisés pour l'immunisation du corps humain ou animal à l'encontre d'un antigène, d'agents pathogènes, tels que des virus ou des parasites. La partie peptidique peut notamment être un fragment de la protéine codée par le gène ENV des virus HIV, en particulier le fragment 312-327 ou le fragment 302-336 de cette protéine.

## Description

La présente invention a pour objet de nouveaux lipopeptides inducteurs des lymphocytes T cytotoxiques.

Elle a d'autre part pour objet l'utilisation de tels lipopeptides comme vaccins.

La plupart des vaccins utilisés induisent une réponse par l'intermédiaire des anticorps. Néanmoins , il a été montré que les lymphocytes cytotoxiques peuvent de manière efficace protéger des souris contre divers microorganismes pathogènes (Skehel et ai, Proc. Natl. Acad. Sci. USA. 1982, 79; 968 ; Lukacher al, Exp. Med. 1984 , 16O: 814). Ceci a été aussi montré pour des lymphocytes T cytotoxiques humains dirigés contre des cytomégalovirus (Quinnan et al., N. Engl. J. Med. 1982, 3O7: 7, Rook et al , Am. J. Med. 1984, 76: 385). Cependant peu de choses sont connues concernant l'induction de l'immunité due aux lymphocytes.

Des auteurs ont tenté d'induire in vivo des lymphocytes T cytotoxiques (CTL) à l'aide de peptides dérivés de l'ovalbumine (Carbone et al. J. Exp. Med. 169: 6O3, Ishioka et al. 1989, J. Immunol. 143:1O94). Ces auteurs ont obtenu des immunisations, mais ces résultats sont spécifiques des peptides dérivés de l'ovalbumine .

AICHELE et al. ( 1990) J. Exp. Med. 171: 1815) ont , quant à eux réussi à induire une réponse T cytolytique par des injections répétées in vivo d'un peptide synthétique en émulsion dans de l'adjuvant incomplet de Freund . Ces auteurs n'indiquent pas l'importance de l'adjuvant dans leurs conditions d'immunisation. Cependant, ils laissent supposer qu'un adjuvant est nécessaire à l'obtention d'une telle réponse .

La demande EP-2O3.676 a pour objet un vaccin destiné à induire une réponse par l'intermédiaire des cellules T , comprenant des conjugués peptide-acide gras. L'acide gras utilisé est l'acide palmitique. Cependant , ce vaccin comprend aussi de l'adjuvant de Freund .

A la connaissance du demandeur , seuls DERES et al. (Nature, Volume 342, 3O Novembre 1989) ont décrit l'utilisation d'un lipopeptide synthétique pour induire in vivo les lymphocytes T cytotoxiques (CTL) . Dans cet article , le fragment NP 147-158 d'une nucléoprotéine du virus influenza est couplé avec du tripalmitoyl-S-glycéryl cystéinyl-séryl-sérine(P3CSS). Il est montré que le conjugué peptide NP 147-158-lipide P3CSS induit une réponse CTL contre des cellules cibles infectées par le virus influenza, tandis que des souris immunisées avec seulement le peptide NP 147-158 ou avec le peptide Ser Ser-NP 147-158 ne génèrent pas de lymphocytes T cytotoxiques dirigés contre ce virus.

Il est aussi à noter que des lipopeptides ont déjà été utilisés pour induire des réactions immunologiques vis-à-vis d'antigènes précis , mais les réponses engendrées impliquent la synthèse d'anticorps, et non des réponses lymphocytaires T.

HOPP ( Molecular Immunology, 21, l3-16, 1984) a montré que l'on pouvait obtenir des anticorps dirigés contre un déterminant du virus de l'hépatite B en immunisant des lapins à l'aide d'une molécule constituée d'un peptide de l5 acides aminés correspondant au déterminant antigénique du virus de l'hépatite B et d'un résidu pseudolipidique, la dipalmitoyl lysine.

La demande EP-93.851 décrit des lipopeptides comprenant une séquence peptidique de 6 à 5O acides aminés liée à une partie lipophile telle qu'un acide gras palmitique, stéarique , béhénique, oléique ou mycolique . Il est mentionné que ces lipopeptides induisent la synthèse d'anticorps.

L'article de Wiesmüller et al (Vaccine, vol.7, n°1, 29-33, l989) décrit l'utilisation d'un lipopeptide formé d'une partie de la séquence du virus FMDV ( VP₁) et du lipide P₃CSS pour induire la synthèse d'anticorps.

Le résumé de l'article de Jacob et al (Chemical Abstracts, vol.1O4, n°21, 472, abrégé 184.455 x, 1986) concerne l'induction de la synthèse d'anticorps par un lipopeptide formé de la toxine tétanique liée à un résidu dipalmityl.

Le résumé de l'article de Watari et al (Chemical Abstracts, vol. 1O6, p 516, résumé n° 154.381 u, 1987) est relatif à l'utilisation de peptides correspondant à la région N-terminale de la glycoprotéine D du virus HSV couplés à l'acide palmitique . Il est indiqué clairement dans cet article qu'il y a induction d'une réponse par l'intermédiaire des cellules T mais que cette réponse n'est pas due à des lymphocytes T cytotoxiques .

Deux autres documents concernent la synthèse et l'étude de la structure de lipopeptides .

La demande Internationale WO 89 1O 348 a pour objet des lipopeptides dérivés d'acides gras tels que les acides aminoeicosanique, aminodécanoïque, aminotétradécanoïque, bromodécanoïque et bromododécanoïque.

Il est mentionné que ces composés peuvent être utilisés comme adjuvants et support pour vaccins, mais sans donner de moyens de mise en oeuvre.

Le résumé de l'article de Mercy et al (Chemical Abstracts, vol.1O6, n°25, 264, abrégé n° 2O9.643 p, 1987) concerne l'analyse structurale d'un lipopeptide formé d'un fragment d'une protéine du virus G et du lysine-palmitoyl.

Cette analyse de l'état de la technique montre donc qu'aucune technologie applicable à différents types de déterminants antigéniques n'a été mise au point permettant d'obtenir l'induction des lymphocytes T cytotoxiques, avec une réponse induite forte, et ne nécessitant pas d'administration d'adjuvant .

Le demandeur a montré de manière surprenante que l'on pouvait induire chez un organisme hôte une réponse des lymphocytes T cytotoxiques contre un antigène en immunisant ledit organisme avec un complexe lipopeptidique contenant un des déterminants de cet antigène .

De manière encore plus surprenante, le demandeur a montré que cette induction pouvait être obtenue pour un grand nombre de déterminants antigéniques de divers agents pathogènes.

La présente invention a donc pour objet un lipopeptide comprenant une partie peptidique ayant entre lO et 4O et préférentiellement entre lO et 2O acides aminés environ et comportant au moins un déterminant antigénique , ledit lipopeptide comprenant également une ou plusieurs chaînes dérivées d'acides gras comprenant entre lO et 2O atomes de carbone et/ou un ou plusieurs groupements stéroïdes, modifiés couplés sur des fonctions NH₂-α ou NH₂-ε desdits acides aminés.

Lesdits dérivés d'acides gras peuvent être notamment l'acide 2-amino hexadécanoïque (D,L) de formule (I) suivante : 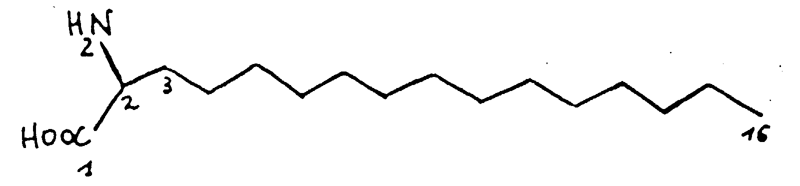 la N-ε-palmitoyl-lysine (L) de formule (II)suivante: 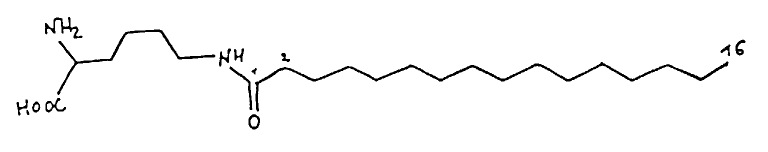 - ou son dérivé de formule : 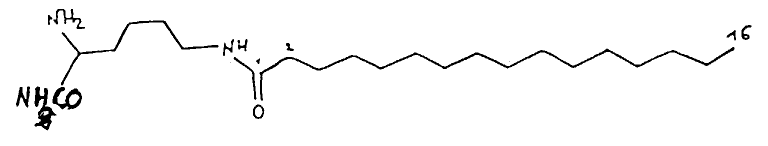 la N,N-diplamitoyl-lysine (L) de formule (III) suivante: 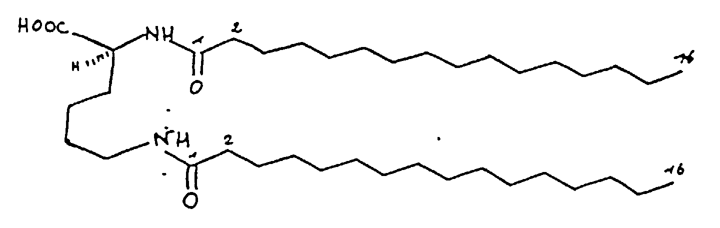 le pimélautide de formule (IV) suivante : 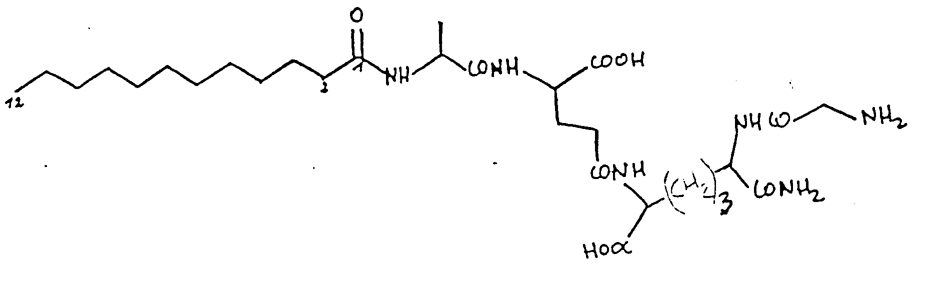 le triméxautide de formule (V) suivante : 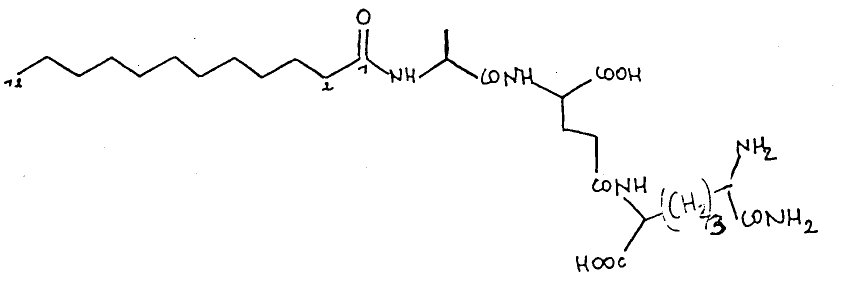 ou un de leurs dérivés.

Lesdits groupements stéroïdes peuvent être la N-ε-[(cholest-5-ényl-3-oxy)-acétyl]-lysine (L) de formule (VI) suivante : 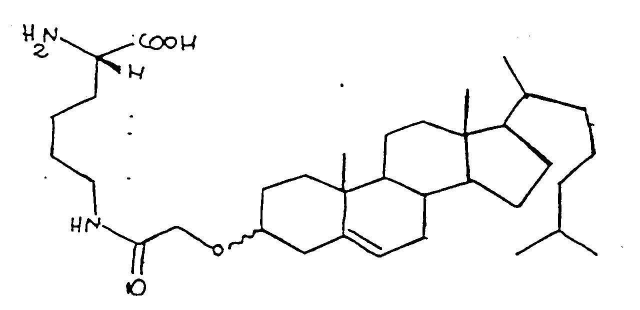 l'acide (cholest-5-ényl-3-oxy) acétique de formule (VII) suivante : 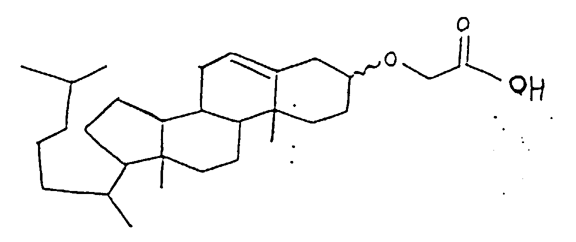 ou un de leurs dérivés.

La partie peptidique peut être un fragment de toute protéine issue d'agent pathogène présentant un déterminant antigénique.

De telles protéines peuvent notamment être les protéines du virus HIV1 ou HIV2, en particulier la protéine codée par le gène env. Dans ce cas, on peut de manière avantageuse utiliser les fragments 312-327 ou 3O2-336 selon la séquence HIV₁-BRU ( Hyers, C.A.B. Rabson, S.F. Josephs, T.F. Smith and E. Wong-Staal (Eds.). 1989. Human retrovirus and AIDS. Los Alamos Laboratoy II:59) de cette protéine, pour former la molécule conjuguée lipopeptidique.

La présente invention a d'autre part pour objet des vaccins à l'encontre de virus ou de parasites contenant l'un des lipopeptides précédemment décrits , et en particulier des vaccins à l'encontre des maladies liées aux virus HIV, lesdits vaccins contenant avantageusement un fragment de la protéine produit du gène env.

La présente invention a de plus pour objet des compositions pharmaceutiques contenant une quantité efficace d'au moins un des composés précédemment décrits en association avec un ou plusieurs diluants ou adjuvants compatibles et pharmaceutiquement acceptables Ces compositions sont en particulier destinées au traitement des maladies liées aux virus HIV par induction des lymphocytes T cytotoxiques .

La présente invention a encore pour objet l'utilisation des lipopeptides précédemment décrits pour l'immunisation du corps humain ou animal à l'encontre d'agents pathogènes par induction des lymphocytes T cytotoxiques . De tels agents pathogènes peuvent être des virus ayant une dépense cytotoxique importante , en particulier les virus HIV1 et HIV2, et certains parasites.

Lesdits lipopeptides peuvent aussi être utilisés contre certains cancers afin d'induire une réponse CTL spécifique de certaines cellules tumorales.

Les lipopeptides objets de la présente invention peuvent être obtenus a partir des constituants protéiques et pseudolipidiques par des méthodes connues de l'homme du métier , en particulier soit par couplage des acides aminés composant la partie peptidique avec le pseudo-lipide immobilisé sur une résine, c'est-à-dire par synthèse en phase solide, soit par couplage du pseudo-lipide sur un peptide immobilisé en phase solide.

Il est à remarquer que les lipopeptides selon l'invention, présentent l'avantage notable de pouvoir être adaptés à l'induction de lymphocytes T cytotoxiques dirigés contre tout type de déterminant antigénique de tout agent pathogène.

A titre subsidiaire la présente invention concerne aussi les intermédiaires de synthèse suivants:
l'acidee 2-tertiobutyloxycarbonylamino hexadécanoïque (D,L) de formule (VIII) suivante : 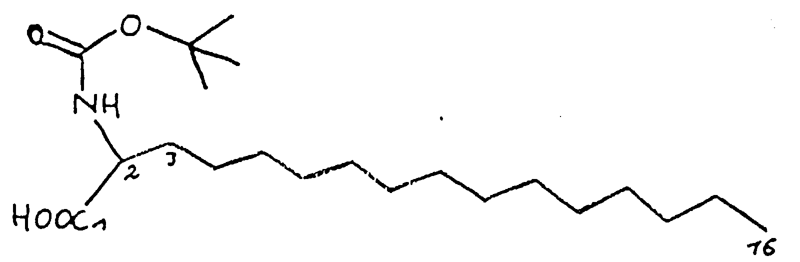 la N-α-terbutyloxycarbonyl ε-palmitoyl-lysine (L) de formule (IX) suivante : 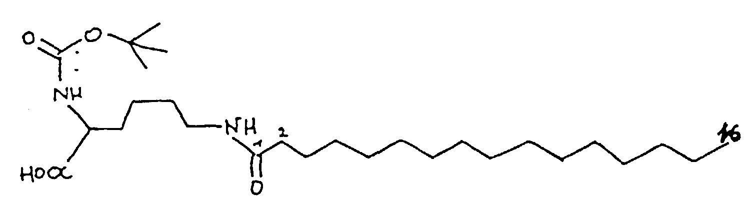 la N-α-fluorénylméthyloxycarbonyl ε-palmitoyl-lysine (L) de formule (X) suivante : 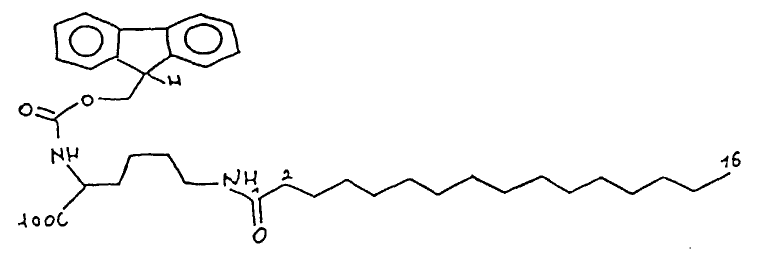 la N-α-tertiobutyloxycarbonyl ε-[(cholest-5-ényl-3-oxy)-acétyl] -lysine (L) de formule (XI) suivante : 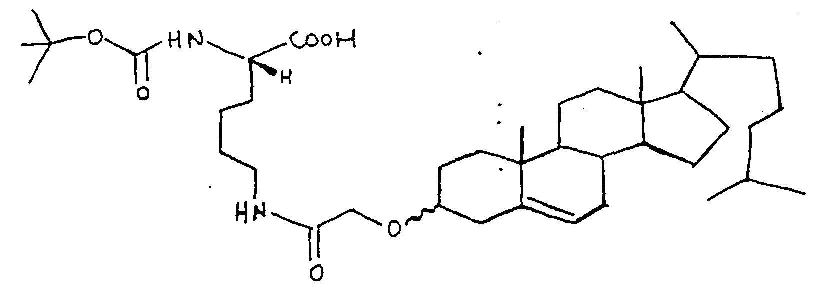 ou la N-α-fluorénylméthyloxycarbonyl ε-[(cholest-5-ényl-3-oxy)-acétyl] lysine (L) de formule (XII) suivante: 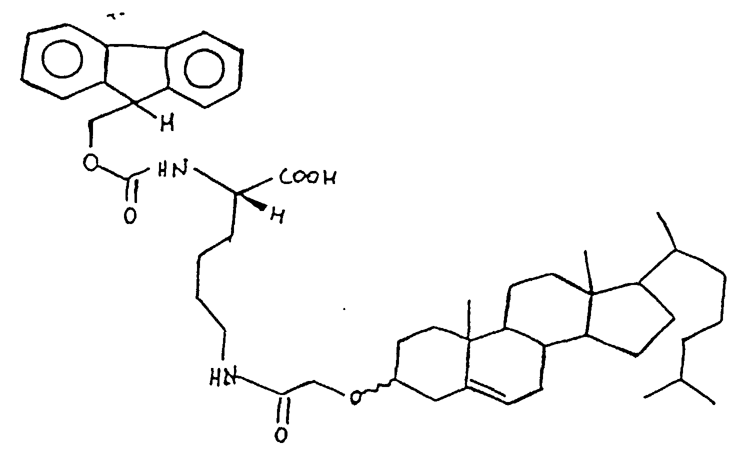 ou un de leurs dérivés.

La présente invention est illustrée sans être aucunement limitée par les exemples d'application suivants dans lesquels les figures l et 3 représentent les spectres en HPLC en phase inverse du lipopeptide V3GP12O, 312-327 succinyl respectivement lors des électrophorèses préparatives et analytiques .

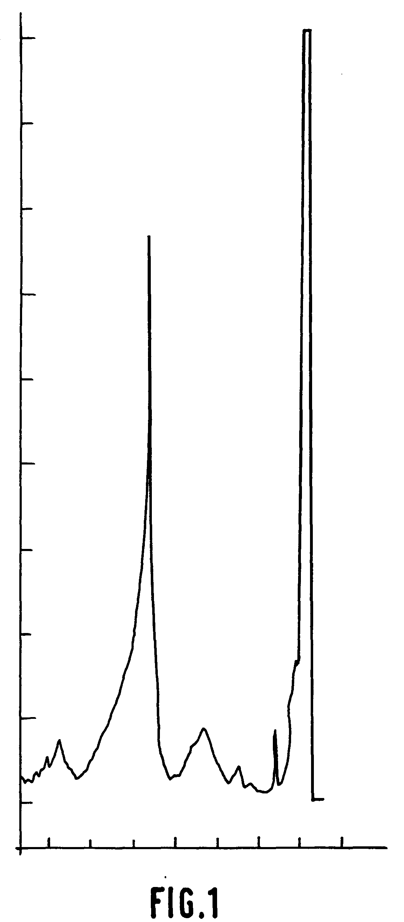

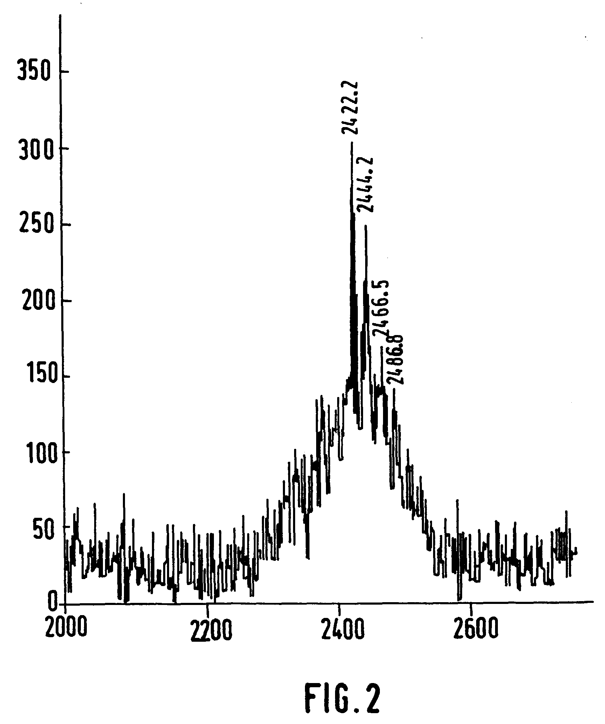

La figure 2 représente quant à elle son spectre de masse .

### EXEMPLE 1.

### Synthèses des résidus pseudo-lipidiques ( ou molécules hydrophobes ).

### l. Synthèse de l'acide t-butyloxycarbonyl amino-2-hexadécanoïque .

### 1.1 Synthèse de l'acide amino-2 héxadécanoïque.

Formule brute : C₁₆H₃₃NO₂
Masse moléculaire : 271,44
Mode opératoire :
   Dans un autoclave préalablement nettoyé à l'acide nitrique 5O% et à l'acide phosphorique lO%, on introduit O,O298 mole d'acide 2-bromohexadécanoïque (lOg) et lOO ml de NH₄OH en solution à 28%. Après agitation, l'autoclave est chauffé à 6O°C pendant l5 heures . Le dérivé aminé en formation précipite dans le milieu réactionnel. Après refroidissement , l'autoclave est lavé , l'eau diffusée et l'éthanol éliminé. Le mélange réactionnel est filtré sur verre fritté de porosité 4. La différence de solubilité des différents produits en milieu éthanol permet d'éliminer les traces d'acide 2-bromohexadécanoïque par rinçages .
Quantité de précipité obtenu : 4,37 g ; rendement de 54%.
Remarque : la non solubilité du dérivé aminé a été vérifiée dans de nombreux solvants ( eau, éthanol, acide acétique à froid, acide formique 7O%, acétate d'éthyle, toluène, toluène/acétonitrile, acétate d'éthyle/acétonitrile). Parmi tous les essais de solubilisation testés, seule l'action d'un détergent spécifique ( le triméthylbenzylammonium hydroxyde) ou de l'acide acétique bouillant a dissous le dérivé aminé.
   Le précipité séché est repris par l5O ml d'acide acétique chauffé à reflux jusqu'à obtention d'une solution limpide jaunâtre . Les pigments colorés sont absorbés sur charbon végétal . Après filtration sur papier filtre plissé, le dérivé aminé purifié est obtenu par cristallisation à partir de l'éluat. Les cristaux blancs obtenus sont récupérés sur verre fritté de porosité 4, lavés à l'acide acétique froid avant d'être séchés en dessicateur sur P₂O₅.
Rendement : 46 % ( le produit étant sous forme acétate on considère un PM de 33O,48).

Le rendement de purification équivaut à 85%.

### 1.2. Synthèse de l'acide T-butyloxycarbonyllamino-2-hexadécanoïque.

- Formule brute : C₂₁H₄₁NO₄
- Masse moléculaire : 371,557.
- Mode opératoire :
   Mise en solution de l'acide amino-2 hexadécanoïque .

A 5 mmoles d'acide aminé sous forme acétate (1.655g) sont ajoutés l.l équivalent de "Triton" (benzyltriméthylammoniumhydroxyde) en solution à 4O% dans le méthanol (2.9 ml) ainsi que lOO ml de DMF. Le mélange réactionnel est laissé sous agitation à température ambiante jusqu'à mise en solution complète. Le DMF est alors évaporé à la pompe à palettes . Le résidu est séché en dessicateur sur P₂O₅.

Protection de la fonction amine .

Le résidu sec est dissous dans un mélange constitué de 36 ml d'eau , 8 ml de KHCO₃ lN et 3O ml d'alcool tertiobutylique. A cette solution, sont ajoutés 2,5 équivalents de terbutyldicarbonate (PM= 218,25). Le pH est ajusté entre 8 et 9 à l'aide d'une solution de Na₂CO₃ lN et maintenu constant dans les premières heures de réaction. La cinétique de couplage est contrôlée par chromatographie sur couche mince de silice .

Après évaporation sous vide de l'alcool tertiobutylique , le produit est repris dans lOO ml d'eau. La phase aqueuse est acidifiée à pH 3 par une solution d'HCl lN. Le Boc-acide aminé est extrait à l'aide d'acétate d'éthyle ( 2 x lOO ml) . La phase organique est lavée à l'eau distillée, séchée sur Na₂SO₄ anhydre, filtrée puis concentrée à l'aide de l'évaporateur rotatif . Le résidu huileux est additionné de 4 ml d'hexane . La cristallisation du Boc-acide aminé est favorisée par refroidissement en chambre froide . Les cristaux blancs sont récupérés sur verre fritté de porosité 4 , lavés à l'hexane et séchés en dessicateur sur P₂O₅.
Rendement : l6 à l8%.

### l.3. Purification et caractérisation.

### l.3.1. Purification.

Des recristallisations successives ont permis d'augmenter la pureté (augmentation du point de fusion) . La purification a conduit à une baisse de rendement d'au maximum 2% pour l'acide aminé hexadécanoïque et d'au maximum l% pour l'acide aminé protégé.

### l.3.2. Caractérisation.

### A.Point de fusion :

| Produit | Point de fusion obtenu |
|---|---|
| Acide 2-bromohexadécanoïque | 56°C |
| Acide amino-2 hexadécanoïque | 144°C |
| Acide tert-butyloxycarbonyl amino-2 hexadécanoïque | 85°C. |

### B. Chromatographie sur couche mince de silice.

Les solutions ( lO à 2Oµl d'une solution à l mg/ml) sont déposées sur couche mince de silice (Merck gel de silice 6O sans indicateur de fluorescence).

L'acide 2-bromohexadécanoïque est mis en solution dans l'éthanol , l'acide aminé 2-hexadécanoïque dans l'acide acétique bouillant, et le tert-butyloxycarbonylamino-2-hexadécanoïque dans le dichlorométhane .

Choix du solvant de migration.

Les différents systèmes choisis sont :
Système A: butanol/acétate d'éthyl/acide acétique/eau dans les proportions volume/volume : 1/1/1/1.
Système B: acétate d'éthyl/pyridine/acide acétique/eau dans les proportions v/v: 5/5/1/3.
Système C: chloroforme/méthanol/acide acétique dans les proportions v/v : lO/l/o,l.

Révélation .

Après migration, dans le système de solvants appropriés , les couches minces sont séchées 15 minutes à 12O°C avant d'être révélées après aspersion d'un réactif de révélation.

- Le réactif à la ninhydrine spécifique des fonctions amines primaires permet la révélation de l'acide aminé hexadécanoïque non protégé mais aussi du Boc acide aminé, l'aspersion d'acide acétique à 2O% suivie d'un séchage à 12O°C permettant le déplacement du groupement Boc .

L'aspersion à l'aide d'un réactif composé de 2Og de (NH₄)₂SO₄, de 3 ml de H₂SO₄ et lOO ml d'H₂O permet la révélation simultanée des trois produits. Pour cette technique , le séchage de la chromatographie sur couche mince après aspersion, est réalisé à l'aide d'un épiradiateur ( résistance en porcelaine avec rayonnement infra-rouge ).

### Résultat :

| Produit | Solvants | Rf |
|---|---|---|
| Acide 2-bromohexadécanoïque | Système A | O,5 |
| | Système B | l |
| | Système C | l |
| Acide amino-2 hexadécanoïque | Système A | O,82 |
| | Système B | O,94 |
| | Système C | O |
| Acide tert-butyloxycarbonyl amino-2 hexadécanoïque | Système A | l |
| | Système B | l |
| | Système C | O,67 |

### C. La spectrométrie de masse (PDMS BIO-ION 2O). Spectre de l'acide tert-butyloxycarbonyl amino-2 hexadécanoïque.

| MM (g) | (M-H)⁻ | Fragments: |
|---|---|---|
| MM théorique | 37O,557 | |
| MM expérimentale | 37O,8 | 27O |

L'ion moléculaire expérimental et l'ion moléculaire théorique ont une masse identique . L'ion moléculaire se fragmente ; il y a perte du groupement Boc ( pic à 27O) . Le pic 296,6 représente l'ion de masse 27O additionné du groupement CN (nitrocellulose).

### 2. Synthèse du 3-β-(2'carboxyméthoxy) cholest-5-ène.

### 2.1. Synthèse du tosylate de cholestéryle.

- Formule brute : C₃₄H₅₃SO₃,
- Masse moléculaire : 54O,83,
- Mode opératoire :
   Après dissolution de 25,86 mmoles de cholestérol (lOg) dans un minimum de pyridine ( 5 à lO ml), on ajoute un excès de chlorure de tosyle (lOg, 52,6 mmoles). Après agitation pendant l2 heures, la pyridine est éliminée par évaporation à sec. Le résidu est solubilisé dans 2O ml d'acétone à chaud (la température est maintenue inférieure à 55°C pour éviter la formation d'une huile ) . On filtre . Le tosylate de cholestérol est obtenu par cristallisation à partir de l'éluat. Les cristaux blancs obtenus sont lavés à l'acétone froid et séchés en dessicateur sur P₂O₅.
   Rendement : 82 à 85%.

### 2.2. Synthèse du 3β-(2'hydroxyéthoxy)-cholest-5-ène.

- Formule brute : C₂₉H_{5O}O₂.
- Masse moléculaire : 43O,71.
- Mode opératoire:
   A l7,5 mmoles de tosylate de cholestéryle (lOg) dissous dans l2O ml de dioxanne, sont additionnés 3O ml d'éthylèneglycol (480 mmoles) . Le milieu réactionnel est chauffé à reflux pendant 4 heures à 12O°C. Après refroidissement , il est repris dans l5O ml d'eau distillée . Le dérivé alcool formé est extrait à l'éther diéthylique ( 3 x 2OO ml) . La phase éthérée est lavée successivement par du NaHCO₃ 5% ( 2 x 2OO ml) et de l'eau distillée (2 x 2OO ml). Après séchage sur Na₂SO₄ anhydre, la solution éthérée est concentrée jusqu'à obtention d'une huile. Après addition de 4 ml d'hexane, la cristallisation est amorcée par frottement et refroidissement en chambre froide (4°C) . Les cristaux blancs sont récupérés sur verre fritté de porosité 4 et lavés à l'hexane avant d'être séchés en dessicateur sur P₂O₅.
   Rendement : 32 à 34 %.

### 2.3 . Synthèse du 3β-(2'-carboxyméthoxy)-cholest-5-ène.

- Formule brute : C₂₉H₄₈P₃,
- Masse moléculaire : 444,69.
- Mode opératoire :
   On réalise préalablement la solution oxydante : celle-ci comprend 2,67 g d'anhydride chromique, 2,3 ml de H₂SO₄ concentré, le volume étant porté à lO ml avec de l'eau distillée . Le milieu oxydant est ajouté goutte à goutte à 4,66 mmoles (2g) de 3 β-(2'-hydroxyéthoxy)-cholest-5-ène dissous dans 5O ml d'acétone . L'évolution de la réaction est contrôlée par chromatographie sur couche mince. La réaction achevée, le milieu réactionnel est repris dans 25O ml d'eau distillée . Le dérivé acide est extrait à l'acétate d'éthyle ( 3 x 2OO ml). La phase organique est lavée à l'eau distillée ( 2 x 2OO ml), séchée sur Na₂SO₄ anhydre et concentrée jusqu'à obtention d'une huile . L'huile est additionnée de 4 ml d'éther de pétrole . On favorise la cristallisation du dérivé acide par refroidissement en chambre froide (4°C) . Les cristaux blancs sont récupérés sur verre fritté de porosité 4, lavés à l'aide d'éther de pétrole et séchés en dessicateur sur P₂O₅.
   Rendement: 29 à 31 %.

### 2.4.Purification et caractérisation.

### 2.4.1. Purification.

Le p-toluènesulfonate de cholestéryle est purifié par recristallisations successives dans l'acétone. Le β-(2'-hydroxyéthoxy)-cholest-5-ène et le dérivé acide ont tous deux été purifiés par chromatographie sur couche épaisse de silice. A. Chromatographie sur couche épaisse de silice.

Les dépôts sont effectués sur couche épaisse de silice (Merck gel de silice 6O PF ₂₅₄ avec indicateur de fluorescence), les tâches étant révélées par des ultraviolets.

Une solution contenant O,25O mg de produit est déposée sur la plaque de silice, les produits ont tous deux été dissous dans le dichlorométhane .

| Produit | Solvant | Rf |
|---|---|---|
| 3β-(2'-hydroxyéthoxy)-cholest-5-ène | Ether de pétrole/Ether éthylique Proportions volume/volume:l/l | O,48 |
| 3β-(2'-carboxyméthoxy)-cholest-5-ène | Ether de pétrole/Ether éthylique/méthanol Proportions v/v: lO/lO/3 | O,52 |

Les deux produits ont été extraits de la silice avec du méthanol . On observe pour chacun des produits une perte équivalente à environ 3O% de la quantité déposée .

### 2.4.2. Caractérisation.

### A. Point de fusion.

| Produit | Point de fusion (littérature) | Point de fusion |
|---|---|---|
| Paratoluène sulfonate de cholestéryle | 131,5°C-132,5°C | 128°C |
| 3β-(2'-hydroxyéthoxy) | 1O2°C-1O4°C | 99°C |
| 3β-(2'-carboxyméthoxy) cholest-5-ène. | 16O°C-161°C | 157°C |

### B .Chromatographie sur couche mince.

Les dépôts (lO à 2O µl) d'une solution l mg/ml sont effectués sur couche mince de silice avec indicateur de fluorescence (Merck Kieselgel 6OF₂₅₄).

La mise en solution des différents produits est effectuée dans le dichlorométhane.

Après migration, dans le système de solvant approprié, les couches minces sont séchées à l'air avant d'être révélées soit par ultra-violet , soit après aspersion avec HClO₄ ( 2O%) et séchage à l'étuve (12O°C pendant 2O minutes).

### Résultat :

### Différents systèmes de solvants.

Système A: Ether éthylique/Ether de pétrole dans les proportions volume/volume : 1/1.
Système B: Ether éthylique/Ether de pétrole dans les proportions v/v : l/2.
Système C: Ether de pétrole/Ether éthylique/Méthanol dans les proportions v/v : 5/5/1.
Système D: Ether de pétrole/Ether éthylique/Méthanol dans les proportions v/v: lO/lO/3.
Système E: Ether de pétrole/Ether éthylique/Méthanol dans les proportions v/v: 5/5/2.
Système F : Ether éthylique.

| Produit | Système de solvant | Rf |
|---|---|---|
| Cholestérol | Système A | O,54 |
| | Système B | O,3 |
| | Système F | O,95 |
| Paratoluène sulfonate de cholestéryle | Système A Système B Système F | O,85 |
| | | O,62 |
| | | l |
| 3β-(2'-hydroxyéthoxy)-cholest-5-ène | Système A | 0,41 |
| | Système B | O,24 |
| | Système F | O,9 |
| 3β-(2'-carboxyméthoxy)-cholest-5-ène | Système A | O |
| | Système B | O |
| | Système C | O,l |
| | Système D | O,42 |
| | Système E | O,78 |
| | Système F Effet de trainée: | RfO--> O,5 |

### C. Spectrométrie de masse (PDMS).

### Analyse du 3β-(2'-carboxyméthoxy)-cholest-5-ène.

| MM (g) | (M-H)- |
|---|---|
| MM théorique | 443,69 |
| MM expérimentale | 443,1 |

L'ion moléculaire expérimental et l'ion moléculaire théorique ont une masse identique.

### D. Résonance magnétique nucléaire du C13.

L'analyse du spectre du 3β-(2'-carboxyméthoxy)-cholest-5-ène a été effectuée par comparaison avec le spectre RMN C13 du cholestérol.

La mise en solution du cholestérol et du 3ß-(2'-carboxyméthoxy)-cholest-5-ène a été réalisée dans le chloroforme deutéré.

### Spectre du cholestérol.

| Pics | d(ppm) obtenu | attribution | d(ppm)théorique |
|---|---|---|---|
| l | 14O,76O6 | C5 ou C6 | fonction alcène: d(ppm) de lOO)145 |
| 2 | 121,7O64 | C5 ou C6 | |
| 3 | 78,5715 | CDCl₃ | |
| 4 | 76,9981 | CDCl₃ | |
| 5 | 75,4O1O | CDCl₃ | |
| l' à 22' | 71 à ll | | fonctions alcanes. |

### Spectre du 3 β-(2'carboxyméthoxy)-choloest-5-ène.

| Pics | d(ppm) obtenu | attribution | d(ppm)théorique |
|---|---|---|---|
| l | 172,2923 | C2' | fonction acide:d(ppm) de l6O à 185 |
| 2 | 139,8394 | C5 ou C6 | fonction alcène: d(ppm) de lOO à 145 |
| 3 | 122,5233 | C5 ou C6 | fonction alcène fonction éther:d(ppm) de 45 à 8O |
| 5 | 79,2943 | Cl' | + léger déplacement |
| 6 | 78,59O3 | CDCl₃ | |
| 7 | 77,OO89 | CDCl₃ | |
| 8 | 75,4146 | CDCl₃ | |
| 9 à 31 | 65 à ll | | fonctions alcanes. |

### E. Analyse élémentaire .

L'analyse élémentaire du 3β-(2'-carboxyméthoxy)-cholest-5-ène a fourni les résultats suivants:

| | Théorique | Obtenu |
|---|---|---|
| % de carbone | 78,3 | 76,25 |
| % d'hydrogène | lO,9 | lO,9 |
| % d'oxygène | lO,8 | 12,5 |

### EXEMPLE 2 - Synthèse des lipopeptides.

### I.Méthode de couplage de l'acide 2-amino hexadécanoïque.

Le choix des séquences construites sous forme lipopeptidique s'est porté sur la région 312-327 de la gp12O du virus VIH-l LAV -BRU afin d'étudier la réponse T cytotoxique . On a effectué 3 préparations avec cette séquence.

La synthèse a été réalisée en phase solide (MERRIFIELD R.B., 1963, J. Am. Chem. Soc. 85, 2149-221O).

Tous les lipopeptides ont été synthétisés sur une résine benzhydrylamine (chargée à O,72 millimole/gramme). Dans tous les cas , le premier acide aminé greffé est l'acide BOC amino-2 hexadécanoïque ( 2 équivalents).Ceci a permis d'obtenir des constructions où l'acide aminé C-terminal est l'acide amino-2 hexadécanoïque sous forme amide afin d'éviter la présence d'une charge à proximité de la chaîne aliphatique hydrophobe. Après acétylation par l'anhydride acétique en milieu basique, afin de bloquer les sites réactifs libres, on a effectué le clivage du BOC N-terminal puis le couplage du premier acide aminé de la séquence.

Toutes ces étapes ont été réalisées manuellement, ce qui a permis d'effectuer des contrôles très précis des couplages de l'acide aminé pseudolipidique et du premier acide aminé sur celui-ci.

L'activateur de couplage est l'hexafluorophosphate de benzotriazolyl-N-oxytriadiméthylaminophosphonium (BOP) en présence d'hydroxybenzotriazole (HOBt) et de diisopropyléthylamine (DIEA). Grâce à cette méthode de couplage très performante, le rendement de ces deux réactions de couplage a toujours été supérieur à 99,5% malgré le fort encombrement stérique dû à l'acide BOC amino-2 hexadécanoïque.

La suite des synthèses s'est effectuée de façon classique en automatique jusqu'au dernier acide aminé. A ce stade la peptidyl-résine a été divisée en 3 Pots, traités manuellement :
- l lot a été conservé tel quel;
- l lot a été greffé par l'acide BOC amino-2 hexadécanoïque.
   Le couplage manuel (au BOP) de ce dernier a été suivi d'un clivage du BOC N-terminal et d'une acétylation de toutes les fonctions amines ainsi démasquées . Cela a permis d'éviter la présence d'une charge à proximité de la chaîne aliphatique hydrophobe de l'acide aminé pseudolipidique ,
- l lot a été greffé par la diBOC, Nα,ε-lysine. Le couplage manuel ( au BOP) de cette dernière a été suivi d'un clivage des deux BOC et du couplage manuel de l'acide palmitique ( au BOP). Nous avons ainsi obtenu des peptides possédant une dipalmitoyl-lysine en position N-terminale.

Ces couplages effectués manuellement ont fait l'objet d'un contrôle étroit qui a révélé des rendements toujours supérieurs à 99,5%. Ces résultats confirment l'intérêt de l'utilisation du BOP comme agent activant en synthèse peptidique, surtout pour coupler les acides aminés pseudolipidiques ou pour effectuer un couplage sur ces derniers. Les lipopeptides synthétisés ont ensuite été clivés de leur support . Les lipopeptides dérivés de la séquence 312-327 ont été clivés par traitement à l'acide fluorhydrique anhydre. Les rendements de coupure sont assez faibles, compris entre 4O et 7O%.

Il existe au moins deux explications à ces valeurs :
l) le clivage d'un peptide greffé sur une résine benzhydrylamine n'est jamais complet dans les conditions usuelles de coupure;
2) la présence de l'acide amino-2 hexadécanoïque, directement en contact avec la résine, a certainement amplifié ce phénomène.

Après deux lavages (TFA-éther), l'identité des lipopeptides a été contrôlée en analyse d'acide aminé après hydrolyse acide totale et , pour certains, en spectrométrie de masse PDMS. Leur homogénéité a été vérifiée en chromatographie sur couche minche de silice et CLHP en phase inverse analytique.

### II. Méthodes de couplage du pimélautide et du triméxautide.

### l) Méthode de couplage du pimélautide ( ou du triméxautide ) à l'extrémité N-terminale d'un peptide par le moyen d'un maillon succinyle.

Cette méthode s'applique à la fixation du pimélautide ( ou du triméxautide ) non protégé sur un peptide construit en phase solide , encore fixé sur la résine, et aux chaînes latérales protégées .

Le triméxautide et le pimélautide (Rhône Poulenc) présentent tous deux , deux fonctions carboxyliques libres, et une fonction amine primaire libre . La création d'une liaison amide prédéterminée entre le peptide et le piumélautide ( ou le triméxautide), ne peut se faire que par utilisation du pimélautide ( ou de triméxautide) comme partenaire aminé .

La succinylation du peptide sur la résine permet d'en faire le partenaire carboxylique.

### DEPROTECTION

Le groupement terbutyloxycarbonyle, qui protège temporairement l'extrémité N-terminale du peptide sur la résine, est clivé par l'action d'une solution d'acide trifluoroacétique à 4O% dans le dichlorométhane , pendant 2O minutes sous agitation.

La résine est lavée par :
- deux fois 2O ml de dichlorométhane,
- deux fois 2O ml de diisopropyléthylamine à 5% dans le dichlorométhane ,
- deux fois 2O ml de diméthylformamide (pendant 3 minutes pour chaque lavage ).

### SUCCINYLATION.

Elle est réalisée en effectuant trois fois le couplage par mise en présence de la résine de la solution de succinylation avec :
- un quintuple excès d'anhydride succinique ( solution à 5% dans la N-méthylpyrrolidone ).
- de la diisopropyléthylamine en quantité stoechiométrique par rapport aux amines de la résine (pendant 2O minutes sous agitation ).

### ACTIVATION.

L'activation du carboxyle maintenant présent sur la résine est réalisée de la façon suivante :

la résine est soumise à l'action de la solution activante ( pendant 15 minutes à température ambiante, et sous agitation ):
- B.O.P. ( hexafluorophosphate de benzotriazolyloxy trisdiméthylamino phosphonium) : 3 excès par rapport aux carboxyles,
- H.O.B.T. ( hydroxybenzotriazole) : 3 excès par rapport aux carboxyles,
- diisopropyléthylamine : 7 excès par rapport aux carboxyles en solution dans la N-méthylpyrrolidone .

### LAVAGE :

La solution est lavée par :
- 3 fois 3O ml de diméthylformamide,
- 3 fois 3O ml de dichlorométhane.

### COUPLAGE:

La résine est soumise à l'action de la solution de couplage :
- pimélautide ( ou triméxautide): 3 excès par rapport à l'ester activé d'hydroxybenzotriazole,
- diisopropyléthylamine : 3 excès par rapport à l'ester,
- diméthylsulfoxyde lO%
- N - méthylpyrrolidone 90 %: quantité suffisante pour dissoudre le pimélautide ( ou le triméxautide ).

La solution saturée est environ à 4% de pimélautide ou de triméxautide après sonication et passage 2 minutes à 5O°C.

### 2)Synthèse en phase solide du V3GP12O, 312-327 succinyl.

### a) N-protection du pimélautide ( ou du triméxautide) par le groupement tert-butyloxycarbonyle.

5OO micromoles de pimélautide ( ou de triméxautide ) sont dissous dans lO ml d'une solution tampon carbonate O,l M à pH 9,5.

On ajoute lO ml d'une solution de pyrocarbonate de diterbutyle à lOO mmoles/l.

Un pH compris entre 9 et lO est maintenu pendant lOO heures par ajoût de carbonate disodique.

Le milieu réactionnel est ensuite dilué par lO ml d'eau et lO ml d'éther diéthylique, et la phase aqueuse lavée est acidifiée à pH 2,5 par l'hydrogénosulfate de potassium.

Une extraction par lOO ml de dichlorométhane, suivi d'une évaporation du solvant à l'évaporateur rotatif conduit à la cristallisation du Boc-pimélautide ( ou Boc-triméxautide).

L'incorporation du Boc-pimélautide ( ou du Boc-triméxautide ) en synthèse peptidique en phase solide génère deux isomères de position.

### b)CLIVAGE ET PURIFICATION.

Le clivage du peptide en fin de synthèse est réalisé par l'acide fluorhydrique anhydre.

Le peptide est alors purifié par gelfiltration et HPLC préparative en phase inverse de type C₄.

La figure l représente le spectre à 235 nm de l'HPLC préparative , obtenu sur 2O mg de lipopeptide dissous dans HCOOH.

Le lipopeptide obtenu est alors analysé par hydrolyse acide totale, par chromatographie analytique HPLC C₄ et en spectrographie de masse.

La figure 2 représente le spectre de masse. On observe un pic distinc à 2422,2 ce qui correspond à la masse du lipopeptide.

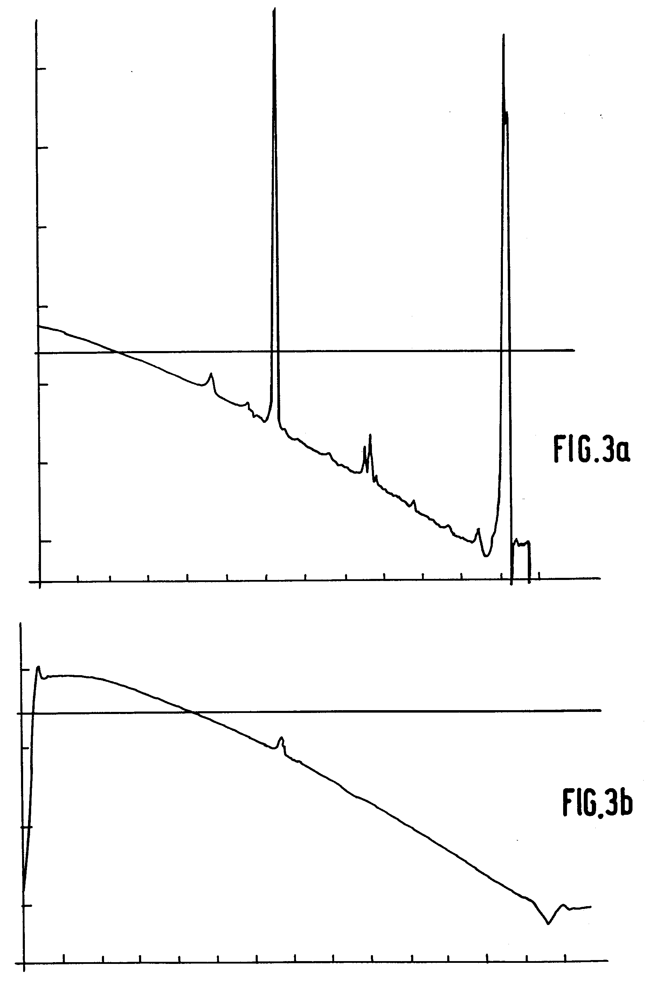

Les figures 3a et 3b représentent respectivement la chromatographie analytique HPLC C₄ du lipopeptide et du témoin sans lipopeptide.

Les conditions de la chromatographie sont les suivantes :
solvant (A) : trifluoroacétate à O,5%o.
gradiant : solvant B: acétonitrile O,75%.
trifluoroacétate à O,5%o.
gradiant de lO% à 8O% en 12O mn.
mesure à une longueur d'onde de 215 nm.
Lors de l'hydrolyse acide totale, l'acide diaminopimélique (Dap) présent dans le pimélautide et le triméxautide constitue un bon marqueur du couplage.

### Résultats de l'hydrolyse acide totale

| Aminoacides | nanomoles | mesuré | théorique | mesuré/théorique |
|---|---|---|---|---|
| Thr | 3.25OO | O.97 | 1 | O.97/1 |
| Glu | 7.1OOO | 2.11 | 2 | l.O6/1 |
| Pro | 3.18OO | O.95 | 1 | O.95/1 |
| Gly | lO.35OO | 3.O8 | 3 | l.O3/1 |
| Arg | 6.6900 | l.99 | 2 | l.OO/1 |
| Val | 3.3900 | l.Ol | l | l.O1/1 |
| Ile | 9.58OO | 2.85 | 3 | O.95/1 |
| Phe | 3.35OO | l.OO | l | l.OO/1 |
| Lys | 3.52OO | l.O5 | l | l.O5/l |
| Arg | 9.92OO | 2.95 | 3 | O.98/1 |
| Dap | 3.5OO | l.O5 | l | l.O5/1 |

### EXEMPLE 3

### Immunisation à l'encontre du peptide NP 147-158.

Les immunisations des souris sont effectuées comme suit :

### Immunisations:

Les souris ont été injectées avec les préparations de lipopeptides par voie intrapéritonéalle ( i.p.) ou par voie sous-cutanée (S.C.), avec ou sans adjuvant .

Il faut au moins deux injections ( espacées de 8 à 3O jours) pour obtenir des CTL.

Chaque injection contient 5 x lO⁻⁸ mole de lipopeptide.

### Détection des CTL

8 à 21 jours après la dernière injection, la rate des souris immunisées a été prélevée, les splénocytes de ces souris ont été cultivés in vitro à raison de 5 x lO⁶ splénocytes/2 ml de milieu de culture classique (DMEM + lO% Fes + pyruvate + acides aminés non essentiels + β-2-Mercaptoéthanol) contenant 5µM du peptide correspondant à celui impliqué dans la construction lipopeptidique.

A partir du 5ème jour de culture in vitro, l'activité des CTL peut être détectée par le test classique de relarguage de ⁵¹Cr. ( Martinon et al, J. Immunol., 142; 3489-3494, 1989).

L'activité des CTL est testée à l'encontre de cellules cibles syngéniques en présence du peptide (NP 147-158 R⁻, P3CSS Pep.NP, ou L1-Pep.NP) ou à l'encontre de cellules cibles infectées par le virus influenza A.

Les résultats obtenus sont résumés dans le tableau I. La première partie du tableau concerne des résultats déjà obtenus, avec le virus influenza total, la protéine NP 147-158 R du virus influenza et le lipopeptide P3 CSS-PEPNP, qui est composé du peptide NP 147-158 couplée à la tripalmitoyl S-glycérylcystéinyle-séryle-sérine (DERES et al. précédemment cité ) .

La seconde partie du tableau concerne d'une part les essais d'immunisation effectués avec des liposomes contenant le peptide NP 147-158, et avec des lipopeptides Ll, L2 et L3. Ces lipopeptides sont des molécules contenant respectivement une partie peptidique (NP 147-158) et une partie lipidique. Les lipopeptides L1, L2 et L3 ont donc les formules suivantes :

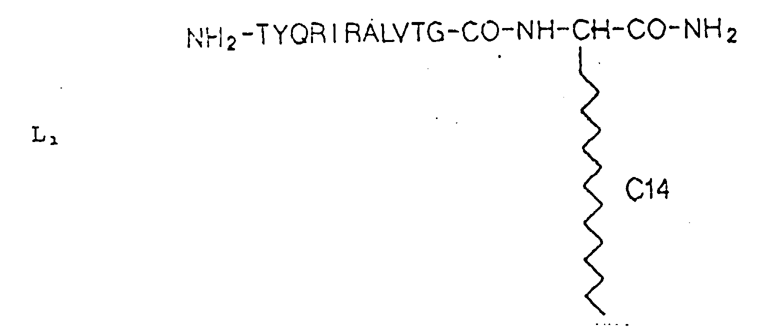

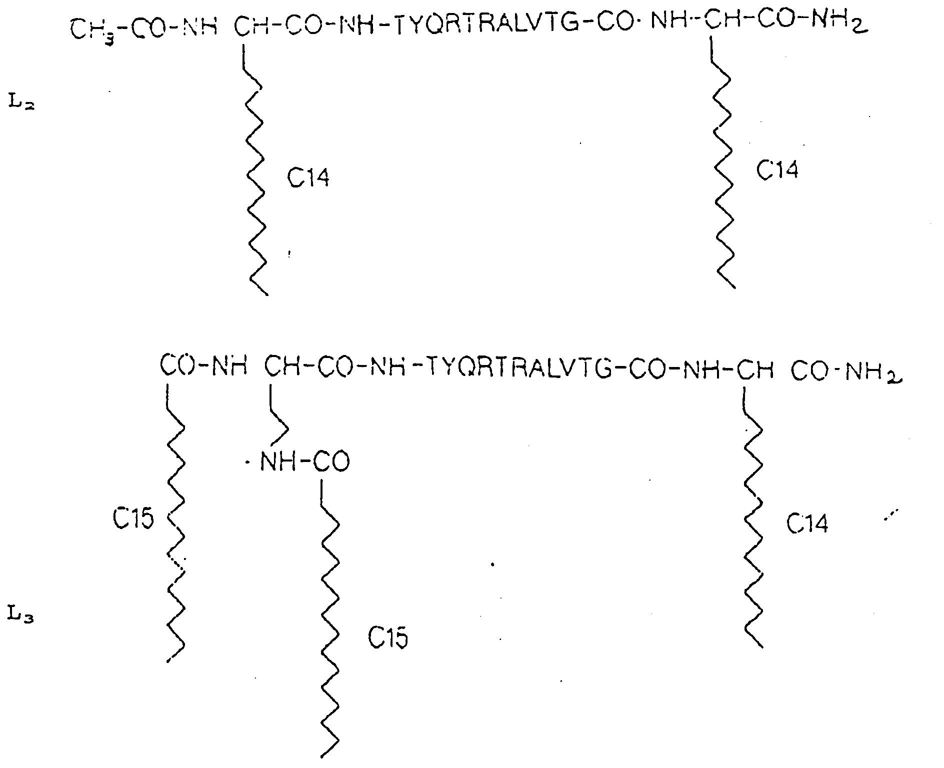

Les activités cytolytiques après 5 jours, l2 jours et plus de 21 jours montrent que l'on obtient une très forte activité pour le lipopeptide L1 comparé aux autres essais effectués.

### EXEMPLE 4

### Immunisation par C₁, CB₂ et CB₃ à l'encontre du peptide ENV 312-327.

Ce peptide est un fragment de protéine, codé par le gène env du virus HIV.

Les protocoles expérimentaux sont identiques à ceux décrits dans l'exemple 3.

Le tableau II résume les résultats obtenus.

Dans ce tableau, CB₁, CB₂ et CB₃ correspondent à des lipopeptides formés à partir du peptide issu de la protéine ENV et d'un lipide.

Les formules de CB₁, CB₂ et CB₃ sont les suivantes :

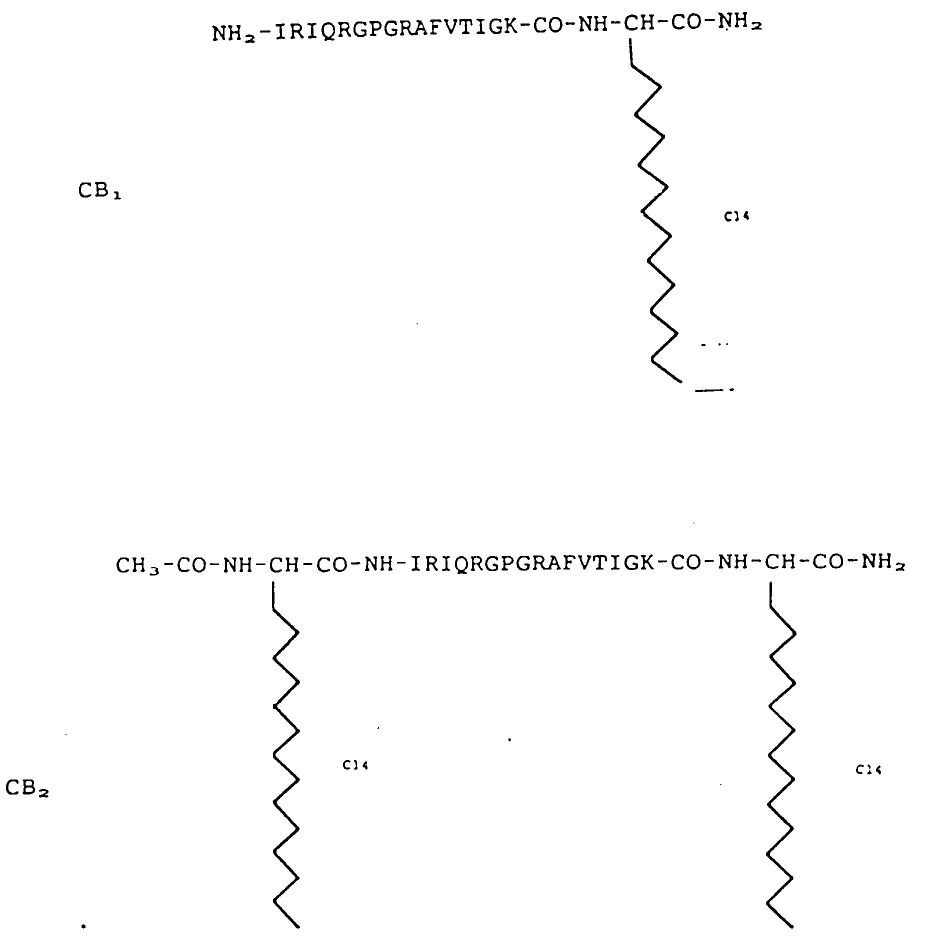

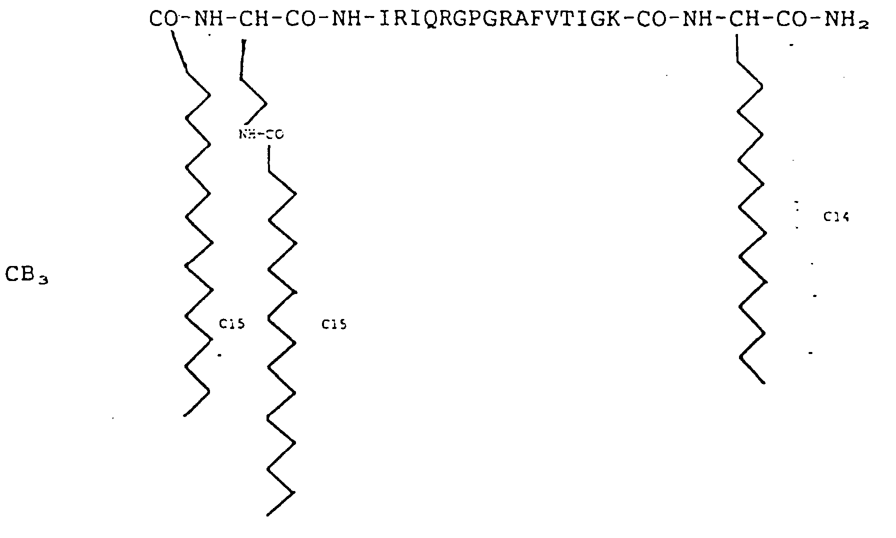

Les résultats du tableau II montrent une activité importante pour l'un des lipopeptides (CB₁).

### EXEMPLE 5:

### Immunisation à l'encontre du peptide ENV 3O2-335.

Ce peptide est le fragment 3O2 à 335 de la protéine ENV du virus HIV.

Les protocoles expérimentaux sont identiques à ceux décrits dans l'exemple 3.

Les résultats sont figurés dans le tableau III.

Les formules des lipopeptides CB₆, CB₇ et CB₈ sont les suivantes:

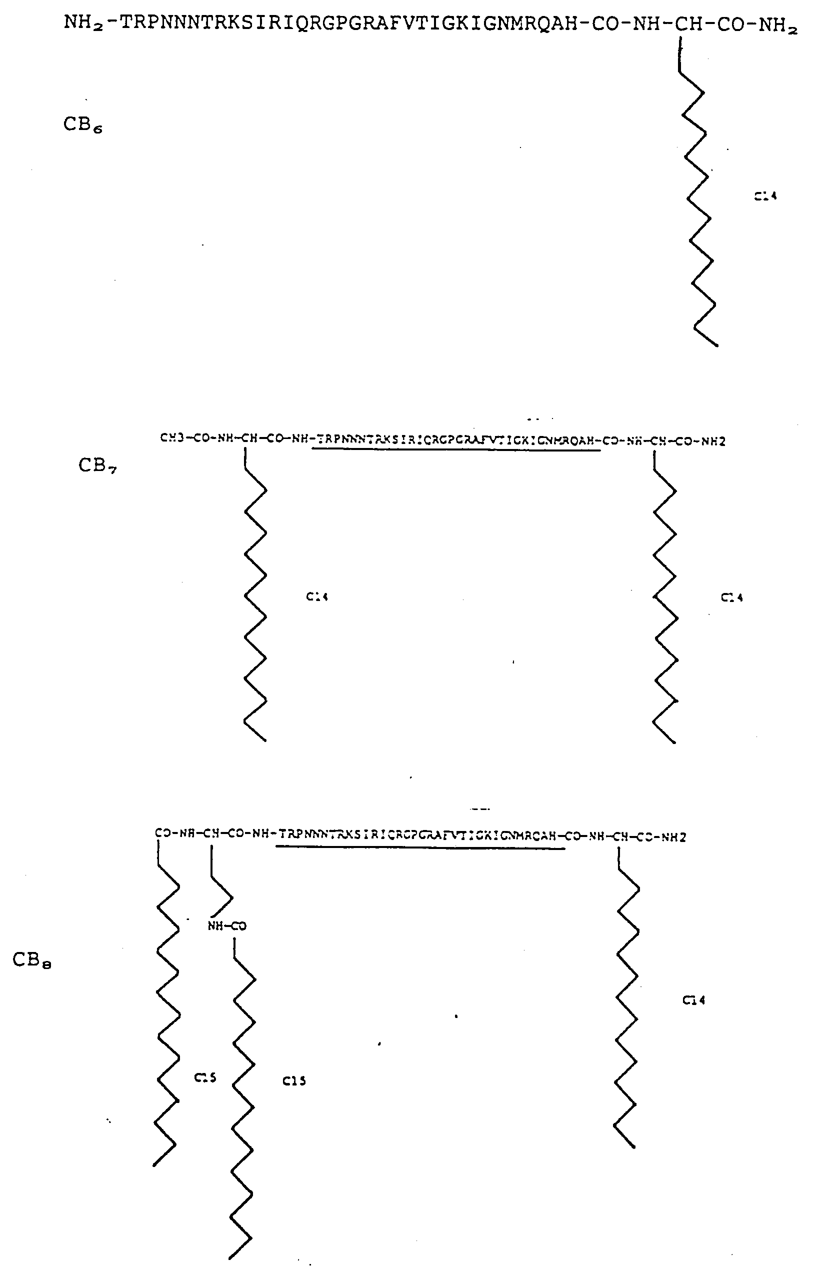

On remarque dans le tableau III que les deux lipopeptides CB₆ et CB₇ montrent des activités cytolytiques très supérieures au témoin.

### EXEMPLE 6:

### Immunisation par CB₁, CB₄,CB₅ CB₁₇, CB₁₉,CB₂₁ et CB₂₅ à l'encontre du peptide ENV 312-327.

Les protocoles expérimentaux sont sensiblement identiques à ceux décrits dans l'exemple 3.

Le tableau IV reprend les résultats obtenus.

La formule de CB1 est indiquée dans l'exemple 4.

Les formules de CB₄,CB₅ CB₁₇, CB₁₉, CB₂₁ et CB₂₅ sont les suivantes :

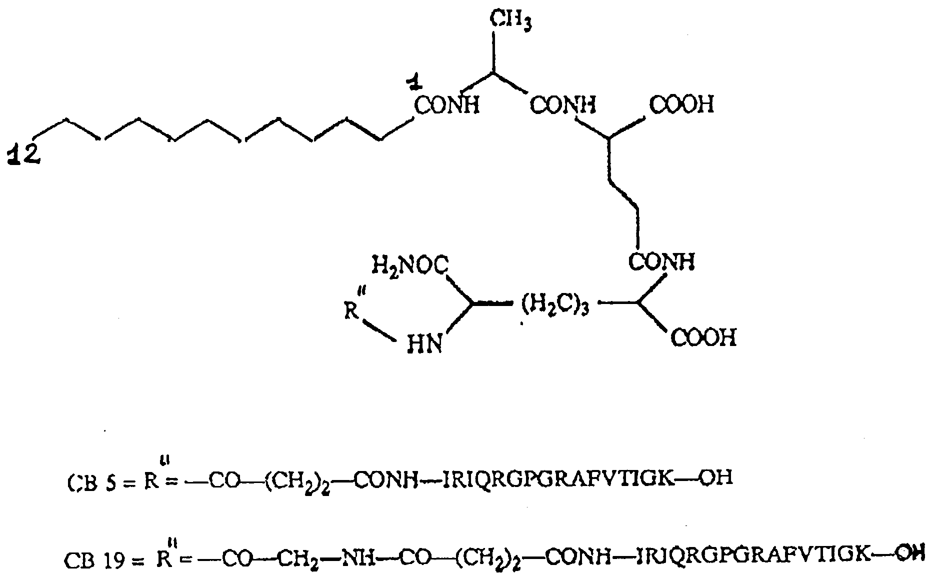

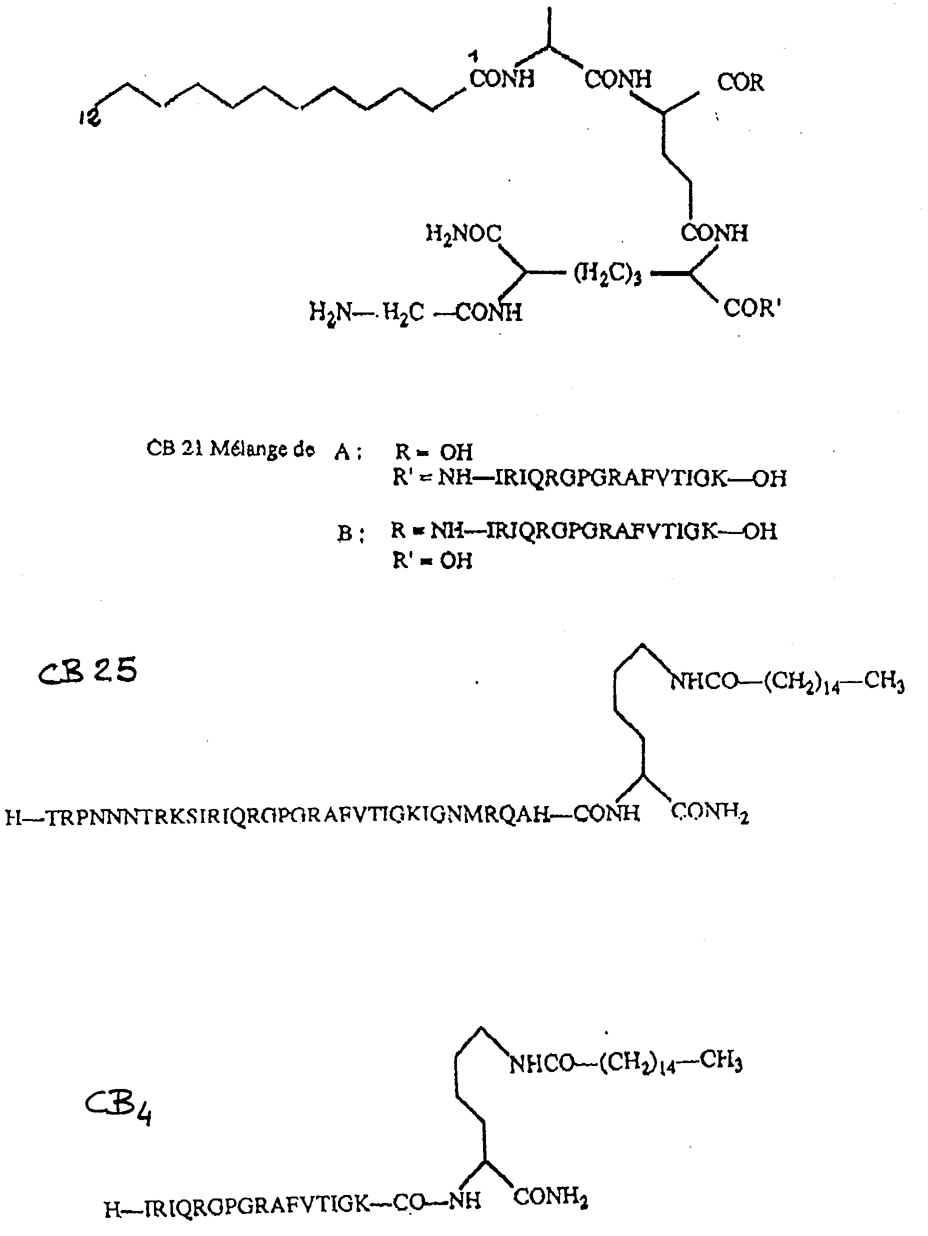

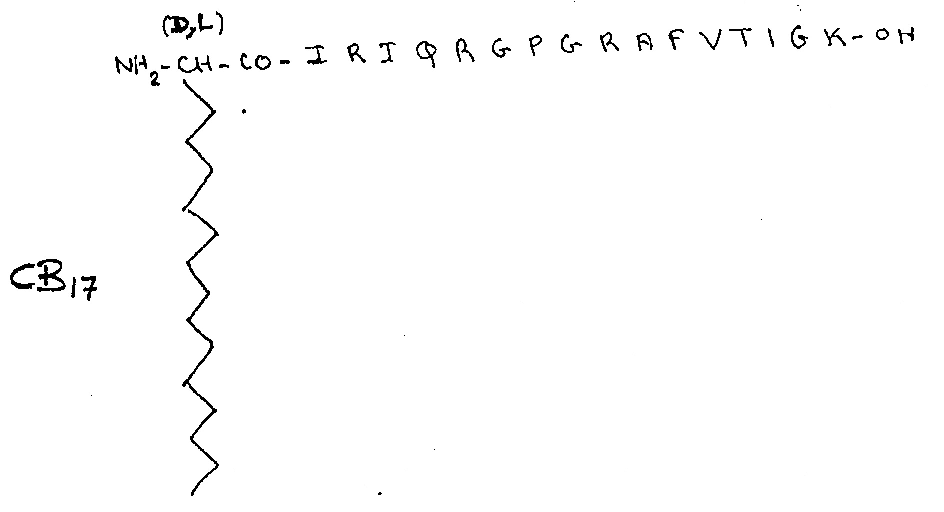

L'extrémité C-terminale de la séquence 312-327 de la protéine GP 12O de HIV-1 (LAV-BRU) est sous forme carboxylique . Un acide a amino hexadécanoïque racémique a été fixé sur la région N-terminale . La fonction N-terminale est sous forme amine.

**TABLEAU I**

| IMMUNISATIONS ANTI-PEPTIDE NP 147-158R (Virus Influenza) | | | | | |
|---|---|---|---|---|---|
| Injection in vivo | Stimulation in vitro | Cible | Activité cytolytique | | |
| | | | j5 | j12 | ≥ j21 |
| O | NP.147-158R⁻ | NP.147-158R⁻(a) | - | - | ++ |
| | | Virus Influ.(b) | | | (+) |
| Virus Influ. | NP.147-158R⁻ | NP.147-158R⁻ | (++) | (++) | (+++) |
| | | Virus Influ. | (++) | (++) | (+++) |
| NP.147-158R⁻ | NP.147-158R⁻ | NP.147-158R⁻ | (-) | - | + |
| | | Virus Influ. | (-) | - | |
| | Virus Influ. | Virus Influ. | (-) | | |
| P₃CSS-Pep.NP | NP.147-158R⁻ | NP.147-158R⁻ | (++) | | |
| | | Virus Influ. | (++) | | |
| | | P3CSS-Pep.NP | (++) | | |

| LIPOSOME-Pep.NP(c) | | | | | |
|---|---|---|---|---|---|
| [1x s.c. Syntex] | NP.147-158R⁻ | NP.147-158R⁻ | - | - | |
| [lx i.p.] | NP.147-158R⁻ | NP.147-158R⁻ | - | ⁻ | |
| [2x s.c. Syntex] | NP.147-158R⁻ | NP.147-158R⁻ | - | ++ | |
| [2x i.p.] | NP.147-158R⁻ | NP.147-158R⁻ | - | ++ | |

| LIPOPEPTIDES-Pep.NP. | | | | | |
|---|---|---|---|---|---|
| L1-Pep.NP.[2x i.p.] | NP.147-158R | NP.147-158R- | - | +++ | |
| | | Virus Influ. | - | | |
| | | L1-Pep.NP. | +++ | +++ | |
| L2-Pep.NP. [2x i.p.] | NP.147-158R⁻ | NP.147-158R⁻ | - | - | |
| | | Virus Influ. | - | - | |
| | | L1-Pep.NP. | - | - | |
| L3-PEP.NP.[2x i.p.] | NP.147-158R⁻ | NP.147-158R⁻ | - | - | |
| | | Virus Influ. | - | - | |
| | | L1-Pep.NP. | - | - | |
| Les rêsultats entre parenthèses sont dèjà publiés. (a) cellules cibles syngéniques en présence de 3µM de peptide NP-147-158R⁻ (b)cellules cibles syngéniques infectées par un virus influenza A (c) liposomes chargés en peptide NP.147-158 R⁻ | | | | | |

**TABLEAU II**

| IMMUNISATIONS ANTI-PEPTIDE ENV.312-327 (HIV-BRU) | | | | | |
|---|---|---|---|---|---|
| Injection in vivo | Stimulation in vitro | Cible | Activité cytolytique | | |
| | | | j5 | j12 | ≥ j21 |
| O | ENV.312-327 | ENV.312-327(a) | - | - | ++ |
| | | Vac-env (b) | - | - | - |
| | CB1 | ENV.312-327(a) | - | - | -(?) |
| Vac-env | ENV.312-327 | ENV.312-327(b) | (++) | | |
| | | Vac-env (b) | (++) | | |
| ENV.312-327 | ENV.312-327 | ENV.312-327(a) | - | - | ++ |
| | | Vac-env (b) | | | |

| LIPOPEPTIDES-ENV.312-327 | | | | | |
|---|---|---|---|---|---|
| CB1 [lx i.p.] | ENV.312-327 | ENV.312-327(a) | - | | |
| CB1 [2x i.p.] | ENV.312-327 | ENV.312-327(a) | +++ | +++ | +++ |
| | | Vac-env (b) | +++ | +++ | +++ |
| CB1 [lx s.c.Syntex] | ENV.312-327 | ENV.312-327(a) | - | | |
| CB1 [2x s.c.Syntex] | ENV.312-327 | ENV.312-327(a) | ++ | | |
| | | Vac-env (b) | ++ | | |
| CB2 [2x i.p.] | ENV.312-327 | ENV.312-327(a) | - | | ++ |
| | | Vac-env (b) | - | | +++ |
| CB3[2x i.p.] | ENV.312-327 | ENV.312-327(a) | - | | |
| | | Vac-env (b) | - | | - |
| (a) cellules cibles syngéniques en présence de 3µM de peptide ENV 312-327 (b) cellules cibles syngéniques infectées par un virus de la vaccine permettant l'expression du gène env. du VIH. | | | | | |

**TABLEAU III**

| IMMUNISATIONS ANTI-PEPTIDE ENV.3O2-335 (HIV BRU) | | | | | |
|---|---|---|---|---|---|
| Injection in vivo | Stimulation in vitro | Activité cytolytique | | | |
| | | | j5 | j12 | ≥ j21 |
| ENV.3O2-336 | ENV.3O2-336 | | | | |
| | ou | ENV.312-327(a) | - | - | |
| | ENV.312-327 | | | | |

| LIPOPEPTIDES-ENV.3O2-335 | | | | | |
|---|---|---|---|---|---|
| | ENV.3O2-336 | | | | |
| CB6 [2x i.p.] | ou | ENV.312-327(a) | ++ | | |
| | ENV.312-327 | Vac-env (b) | | +++ | |
| | ENV.3O2-336 | | | | |
| CB7 [2x i.p.] | ou | ENV.312-327(a) | ++ | | |
| | ENV.312-327 | Vac-env (b) | | +++ | |
| | ENV.3O2-336 | | | | |
| CBS [2x i.p.] | ou | ENV.312-327(a) | - | - | |
| | ENV.312-327 | Vac-env (b) | | - | |
| (a) cellules cibles syngéniques en présence de 3µM de peptide ENV 312-327 (b) cellules cibles syngéniques infectées par un virus de la vaccine permettant l'expression du gène ENV du VIH. | | | | | |

**TABLEAU IV**

| Immunisations anti-peptide ENV. 312-327 par CB₁, CB₄, CB₅, CB₁₇,CB₁₉,CB₂₁ et CB₂₅ | | | | |
|---|---|---|---|---|
| Injection in vivo | Stimulation in vitro | Cible | Activité cytotoxique | |
| | | | <J14 | <J 21 |
| CB1 (2x s.c. ⁾ | ENV 312-327 | ENV 312-327 | +++ | +++ |
| | | Vac-ENV | +++ | +++ |
| | | CB1 | +++ | +++ |
| CB4 ( 2x i.p.) | ENV 312-327 | ENV 312-327 | +++ | +++ |
| | | Vac-ENV | +++ | +++ |
| CB17 ( 2 x i.p.) | ENV 312-327 | ENV 312-327 | - | ++ |
| | | Vac-ENV | - | + |
| CB19 ( 2 x i.p.) | ENV 312-327 | ENV 312-327 | + | ++ |
| | | Vac-ENV | - | + |
| CB21 ( 2 x i.p.) | ENV 312-327 | ENV 312-327 | ++ | +++ |
| | | Vac-ENV | ++ | +++ |
| CB25 ( 2 x i.p.) | ENV 312-327 | ENV 312-327 | +++ | NT |
| | | Vac-ENV | ++ | |
| CB25 ( 2 x i.p.FIA) | ENV 312-327 | ENV 312-327 | ++ | NT |
| | | Vac-ENV | + | |
| CB25 (3 x i.p.) | ENV 312-327 | ENV 312-327 | +++ | NT |
| | | Vac-ENV | ++ | |
| CB25 ( 3 x i.p. FIA) | ENV 312-327 | ENV 312-327 | ++ | NT |
| | | Vac-ENV | ++ | |
| CB25 ( 2 x s.c.) | ENV.312-327 | ENV 312-327 | +++ | NT |
| | | Vac-ENV | ++ | |
| CB25 ( 2 x s.c. FIA) | ENV 312-327 | ENV 312-327 | +++ | NT |
| | | Vac-ENV | +++ | |
| CB25 ( 3 x s.c.) | ENV 312-327 | ENV 312-327 | +++ | NT |
| | | Vac-ENV | ++ | |
| CB25 (3 x s.c. FIA) | ENV 312-327 | ENV 312-327 | +++ | NT |
| | | Vac-ENV | +++ | |
| CB5 ( 2 x i.p.) | ENV 312-327 | ENV 312-327 | ++ | +++ |
| | | Vac-ENV | ++ | +++ |

## Revendications

1. Lipopeptide comprenant une partie peptidique ayant entre 10 et 40 acides aminés environ et préférentiellement entre 10 et 20 acides aminés environ et comportant au moins un déterminant antigénique induisant spécifiquement des lymphocytes T-cytotoxiques, ledit lipopeptide comprenant une ou plusieurs chaînes dérivées d'acides gras comportant de 10 à 20 atomes de carbone choisis parmi (1) l'acide 2-aminohexadécanoïque, (2) le pimélautide et (3) le triméxautide, et/ou un ou plusieurs groupements stéroïdes modifiés et couplés sur des fonctions αNH2 ou εNH2 desdits acides aminés.

2. Lipopeptide selon la revendication 1, caractérise en ce que le groupement stéroïde est la N-ε-[(cholest-5-ényl-3-oxy)-acétyl]-lysine ou l'un de ses dérivés.

3. Lipopeptide selon la revendication 1, caractérisé en ce que le groupement stéroïde est l'acide cholest-5-ényl-3-oxy acétique ou l'un de ses dérivés.

4. Lipopeptide selon l'une des revendications 1 à 3, caractérisé en ce que la partie peptidique est un fragment d'une protéine comprenant au moins un déterminant antigénique des virus HIV1 ou HIV2.

5. Lipopeptide selon la revendication 4, caractérisé en ce que la partie peptidique est un fragment de la protéine codée par le gène ENV et en particulier le fragment 312-327 ou le fragment 302-336

6. Vaccin à l'encontre d'un agent pathogène contenant l'un des lipopeptides selon l'une des revendications 1 à 5.

7. Vaccin selon la revendication 6 à l'encontre des maladies liées aux virus HIV, caractérisé en ce qu'il contient un ou des lipopeptides selon l'une des revendications 4 et 5.

8. Utilisation d'un lipopeptide selon l'une des revendications 1 à 5 pour la fabrication d'un médicament pour l'immunisation du corps humain ou animal à l'encontre d'un antigène, par induction de lymphocytes T-cytotoxiques.

9. Utilisation d'un lipopeptide selon l'une des revendications 1 à 3 pour la fabrication d'un médicament destiné à l'immunisation du corps humain ou animal à l'encontre de cellules tumorales.

10. Utilisation selon la revendication 8, caractérisée en ce que le médicament est destiné à l'immunisation du corps humain ou animal à l'encontre d'agents pathogènes.

11. Utilisation selon la revendication 10, caractérisée en ce que les agents pathogènes sont des virus.

12. Utilisation selon la revendication 11, caractérisée en ce que les agents sont les virus HIV1 et HIV2, auquel cas l'immunisation est effectuée à l'aide des lipopeptides selon l'une des revendications 4 ou 5.

13. Utilisation selon la revendication 10, caractérisée en ce que les agents pathogènes sont des parasites.

14. Composition pharmaceutique, caractérisée en ce qu'elle contient une quantité efficace d'au moins un lipopeptide selon l'une des revendications 1 à 5 en association avec un ou plusieurs diluants ou adjuvants compatibles et pharmaceutiquement acceptables.

15. Composition selon la revendication 14, caractérisée en ce qu'elle est destinée au traitement des maladies liées aux virus HIV, par induction des lymphocytes T-cytotoxiques.

16. Composition selon la revendication 14, caractérisée en ce qu'elle est destinée au traitement de certains cancers, par induction de lymphocytes T-cytotoxiques.
